(19) Europäisches Patentamt
European Patent Office
Office européen des brevets

(11) Publication number : **0 490 582 A1**

# (12) EUROPEAN PATENT APPLICATION

(21) Application number : **91311344.5**

(51) Int. Cl.[5] : **A61K 7/06**

(22) Date of filing : **05.12.91**

(30) Priority : **07.12.90 EP 90313293**

(43) Date of publication of application : **17.06.92 Bulletin 92/25**

(84) Designated Contracting States : **AT BE CH DE DK ES FR GB GR IT LI NL SE**

(71) Applicant : **UNILEVER PLC**
**Unilever House Blackfriars**
**London EC4P 4BQ (GB)**
(84) **GB**

(71) Applicant : **Unilever N.V.**
**Burgemeester s'Jacobplein 1**
**NL-3015 CA Rotterdam (NL)**
(84) **BE CH DE DK ES FR GR IT LI NL SE AT**

(72) Inventor : **Reid, Euan Stuart**
**4 Meadway, Spital, Bebington**
**Wirral L62 2AR (GB)**
Inventor : **Rosser, David Arthur**
**The Sycamores, The Mount, Heswall**
**Wirral L60 4RD (GB)**
Inventor : **Tan-Walker, Ruby Loo Bick**
**16 School Lane, Guilden, Sutton**
**Chester CH3 7ET (GB)**

(74) Representative : **Ford, Michael Frederick et al**
**MEWBURN ELLIS 2 Cursitor Street**
**London EC4A 1BQ (GB)**

## (54) Hair treatment composition.

(57) A hair treatment composition comprising a water-in-oil emulsion, wherein the water phase constitutes 40-95% by weight of the composition and the oil phase 5-60% by weight of the composition, wherein the oil phase comprises from 1-100% based on the weight of the oil-phase of a silicone material having a viscosity of $10^4$ to $10^9$ mPa.s at 25°C.

EP 0 490 582 A1

The present invention relates to a hair treatment composition, in particular to a hair treatment composition which is suitable for the treatment of hair comprising split-ends.

The occurrence of split-ends in hair is often unwanted, because it leads to a dull appearance and less stylability of the hair.

It is known that silicone materials of high viscosity can be used for the split-end treatment of hair. A problem with split-end treatment products, however, is their greasy feel which is less appreciated by the user. Also these products are often difficult to apply evenly to the hair-ends.

It has now been found that a hair treatment product can be formulated which is suitable for the split-end treatment of hair, said product having improved feel and/or improved spreadability over the hair.

The present invention relates to a hair treatment composition comprising a water-in-oil emulsion, wherein the water phase constitutes 40-95 % by weight of the composition and the oil phase 5-60 % by weight of the composition, wherein the oil phase comprises from 1-100 % based on the weight of the oil-phase of a silicone material having a viscosity of $10^4$ to $10^9$ mPa.s at 25°C. Preferably compositions in accordance with the invention also comprise an emulsifying material for stabilising the water-in-oil emulsion.

The oil phase

Hair treatment compositions of the invention comprise from 5-60 % by weight, more preferably from 10-30 % by weight, most preferably 12-25 % by weight, of an oil phase.

The oil phase comprises from 1-100 % by weight based on the oil phase of a high viscosity silicone material, more preferably 2-40 %, most preferably 5-20 %.

The silicone material for use in compositions of the invention can be any silicone material of the required viscosity. For example, polyalkyl siloxanes, polyalkylaryl siloxanes, aminofunctional silicones, polydiorganosiloxanes or mixtures thereof may be used.

Preferably silicone gums are used as the silicone materials. For the purpose of the present invention the term silicone gum denotes polydiorganosiloxanes having a molecular weight of 200,000 to 2,000,000. Examples of suitable silicone gums are for example described in US 4 152 416. Specific examples of suitable silicone gums are polydimethyl or polydiphenyl siloxane polymers.

Silicone materials for use in the hair treatment compositions of the invention have a viscosity of $10^4$ to $10^9$ mPa.s at 25°C, more preferably from $5x10^4$ to $5x10^8$, most preferably from $10^5$ to $5x10^7$ mPa.s. A suitable method for measuring the viscosity is by means of a glass capillary viscometer (cf Dow Corning CTM 0004) or by a Brookfields synchrolectric viscometer (cf Dow Corning CTM 0050).

It is preferred that the oil phase of compositions in accordance with the invention also comprises a carrier or diluent material for the high viscosity silicone material. Often, high viscosity silicone materials are supplied as a dispersion in a carrier or diluent material, for example as a 5-25 % by weight dispersion of the high viscosity silicone in cyclomethicone, linear dimethicone and/or isoparaffin. These dispersions may advantageously be used in the oil phase of the hair treatment products of the invention. Alternatively or additionally the oil phase may comprise further diluents such as for example low viscosity silicones (having a viscosity of say between 0.1 and 1,000 mPa.s, more preferably 0.5 to 500 mPa.s most preferably 0.65-100.), liquid paraffins or methicones and other solvents such as $C_{10}$ to $C_{12}$ isoparaffins such as Isopar L (Esso), polyisobutene such as polysynlane (Nippon Oils and Fats), squalane such as Squalene (J.G. Marthens), branched chain hydrocarbons e.g. Permethyl 99A (Presperse), branched chain light paraffin oils such as Lytol (Witco) or WM1 (BP), mineral oil such as Marchol 82 (Esso) or Carnation Oil (Witco), long chain alkyl alkanoic esters such as decyl oleate (eg. Cetiol V ex Henkel), isopropyl myristate (eg. Estol 1514 ex Unichema) and glyceryl tri(2-ethyl hexanoate) (eg. Myritol GTEH ex Henkel).

It is also possible to employ vegetable and animal oils, provided that branched-chain alkyl groups are present.

Preferably the level of carrier or diluent material for the high viscosity silicone material constitutes from 0-99 % by weight of the oil phase, more preferably 60-98 %, most preferably 80-95 %.

The oil phase of compositions in accordance with the invention also preferably contain an emulsifying agent.

The level of emulsifying agent is preferably 0-20 % of the oil phase, more preferably 1-10 % of the oil phase. Preferred emulsifying agents are low HLB (hydrophiliclipophilic balance) emulsifiers with an HLB of 1 to 7. Especially preferred is the use of mono-, di- or triphosphoricesters of fatty acids, ethers of glycerol and fatty alcohols, esters of glycerol and fatty acids, ethers of polyethoxylates and fatty acids, sorbitan fatty acid esters. Examples of these materials are Hostaphat KO 300N ex Hoechst, Cremophor WO/A ex BASF, Arlacel 987 and Synperonic A2 ex ICI, Imwitor 780K ex Huls, Arlacel 80, 83, 85 ex Atlas, Deca glycerol tetraor octa-oleate ex PVO International and Simulsol 92 by PCMN. Especially preferred is the use of glyceryl monoisostearate and sorbitan

monoisostearate. These emulsifying agents are especially preferred for use in combination with non-silicone oils.

Other preferred emulsifying agents are silicone glycol surfactants.

Suitable silicone surfactants are for example high molecular weight polymers of dimethyl polysiloxane with polyoxyethylene and/or polyoxypropylene side chains, having a molecular weight of from 10,000 to 50,000 and having the structure:

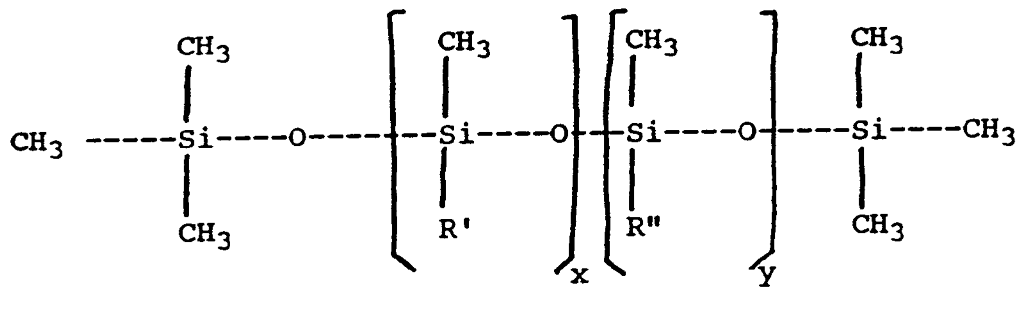

where in the groups R′ are each chosen from -H, $C_{1-18}$ alkyl and R″ is

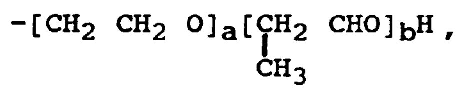

in which
a has a value of from 9 to 115,
b has a value of from 0 to 50,
x has a value of from 133 to 673,
y has a value of from 25 to 0.25.

Preferably, the polymer is an alkoxylated polydimethyl polymer in which:
a has a value of from 10 to 114,
b has a value of from 0 to 49,
x has a value of from 388 to 402,
y has a value of from 15 to 0.75,
the group R″ having a molecular weight of from 1000 to 5000.

A particularly preferred alkoxylated dimethyl polysiloxane polymer is one in which:
a has the value 14,
b has the value 13,
x has the value 249,
y has the value 1.25,

In addition to the high viscosity silicone material, carrier or diluent material (if any) and, preferably, emulsifier, the oil phase may also comprise further ingredients such as for example perfume oils, colouring agents etc.

Preferably the level of perfumes is from 0-5% of the oil phase.

<u>The aqueous phase</u>

Hair treatment compositions of the invention comprise from 40-95 % by weight, more preferably from 70-90 % by weight, most preferably from 75-88 % by weight, of an aqueous phase.

Preferably the aqueous phase comprises 20-100 % by weight of water, more preferred 30-80 %, most preferred 50-70 % by weight,based on the weight of the aqueous phase.

In addition to water, the aqueous phase may for example comprise one or more liquid water-miscible materials. Suitable materials are for example lower alcohols such as ethanol, and polyols such as propylene glycol, glycerol, sorbitol and polyglycerol. Especially preferred is the use of glycerol, which has the additional advantage of providing transparency to the hair treatment composition. Also suitable is the use of polyether materials such as for example polyethyleneglycol or polypropylene glycol having a molecular weight of 100 to 5000, ethoxylated polyols, e.g. Atlas G2330 ex ICI and Glucum E10 ex Amerchol and block copolymers of ethylene

oxide and propylene oxide e.g. Synperonic L42 ex ICI. These materials have the advantage of even further improving the sensory feel of the composition. Other humectants and/or optical index agents may also be used.

Preferably the aqueous phase comprises 0-75 % by weight of liquid water-miscible materials, more preferably 20-65 %, most preferably 30-45 %, based on the weight of the aqueous phase.

Another preferred ingredient for incorporation in the aqueous phase is an electrolyte material, for example selected from water soluble salts such as alkali (earth) metal salts such as sulphates, halogenides, formates, borates, benzoates. and ($C_{1-4}$) tetra-alkyl ammonium halides etc. Water soluble acids such as citric acid, phosphoric acid etc may also be used. The preferred level of electrolyte materials is from 0-25 %, more preferred 1-10 %, most preferred 2-5 %, based on the aqueous phase. Electrolyte materials have the advantage of providing increased stability to compositions of the invention. If the electrolyte material comprises an acidifying agent, this material is preferably included in an amount such that the pH of the aqueous phase is from 4-12, more preferably from 4 to 8, most preferably from 4-6.

## Physical form

Hair treatment compositions according to the invention may take a variety of physical forms, for example they may be liquids, gels, pastes etc. Preferably compositions of the invention are gels having a viscosity of 1,000 to 100,000 mPa.s, more preferably 10,000 to 75,000 mPa.s at 25°C, as measured in a Brookfield RVT viscometer, spindle c, 10 rpm.

## Other ingredients

Hair treatment compositions of the invention may also include minor amounts of other ingredients such as surfactants, antibacterial agents, antidandruff agents, pearlescers, dyes, preservatives, sunscreens, viscosity modifiers, proteins, polymers, buffering agents, herb extracts, oils etc.

## Use

Compositions in accordance with the present invention are preferably used as leave-on products; that is, they are applied to wet or dry hair and are intended to be left on the hair until the hair is washed. Preferably compositions of the invention are applied to recently washed hair which has optionally been dried.

The amount of product which is applied to the hair can vary in a broad range but is preferably 0.1 to 25 mls, more preferred 0.2 to 5 mls.

## Method of preparation

Hair treatment compositions of the invention may be prepared by any suitable method for the preparation of water-in-oil emulsions. A preferred method involves the separate preparation of the oil phase and the aqueous phase by mixing, followed by gradually adding the aqueous phase to the oil phase under stirring.

The invention will be further illustrated by the following examples:

## Example I

The following compositions were prepared as follows:

The aqueous phase and the oil phase were each separately prepared by mixing the ingredients in the listed order, followed by the gradual addition of the aqueous phase to the oil phase under stirring.

The aqueous phase had the following composition:

| Aqueous phase | A | B | C | D | E |
|---|---|---|---|---|---|
| water/citric acid1) | 51.8 | 43.6 | 43.0 | 41.1 | 41.1 |
| glycerol | 40.7 | 46.1 | 46.7 | 49.0 | 19.0 |
| PEG-400 | 5.0 | 7.3 | 7.3 | 7.0 | 7.0 |
| sodium chloride | 1.9 | 2.4 | 2.4 | 2.3 | 2.3 |
| sodium benzoate | 0.6 | 0.6 | 0.6 | 0.6 | 0.6 |

The oil phase had the following composition:

| Oil Phase | A | B | C | D | E |
|---|---|---|---|---|---|
| silicone2) | 64.7 | -- | -- | -- | -- |
| silicone3) | -- | 35.2 | 35.2 | 43.3 | 43.3 |
| diluent4) | 34.8 | 45.5 | 45.5 | 56.0 | -- |
| diluent5) | -- | -- | 18.7 | -- | -- |
| diluent6) | -- | 18.7 | -- | -- | -- |
| diluent7) | -- | -- | -- | -- | 42.0 |
| emulsifier8) | -- | -- | -- | -- | 14.0 |
| perfume | 0.5 | 0.6 | 0.6 | 0.7 | 0.7 |

The relative amounts of aqueous phase and oil phase in the final product were as follows:

| product | A | B | C | D | E |
|---|---|---|---|---|---|
| water phase | 79.9 | 82.4 | 82.4 | 85.7 | 85.7 |
| oil phase | 20.1 | 17.6 | 17.6 | 14.3 | 14.3 |

1) mixture of water and citric acid such that the pH of the water phase is 5.0

2) TP504 ex Union Carbide (10 % of high viscosity silicone gum in cyclomethicone)

3) A74 ex Toray ( 21 % high viscosity silicone gum in dimethicone and isoparaffin)

4) 3225C ex Dow Corning, a 10 % silicone glycol surfactant in cyclomethicone DC344

5) DC 344 ex Dow Corning, a 70/30 mixture of cyclomethicone D4 and D5

6) DC 200/10 a dimethicone having a viscosity of 10 mPa.s

7) Isopar L ex Esso, an isoparaffin

8) Arlacel 987 ex ICI a sorbitan monoisostearate

The products had a non-greasy feel and were easily applicable, spreadable products. The isolated non-emulsified oil phase has a much more greasy feel and was not so easily spreadable over the hair and was more difficult to wash from the hair. A further surprising benefit is that firmp gels were formed even at the low levels of oil phase as used. The viscosities of the prosucts as measured in a Brookfield viscometer were as follows:

| Composition | viscosity (mPa.s) |
|---|---|
| A | 65,000 |
| B | 60,000 |
| C | 33,500 |
| D | 80,000 |
| E | 15,000 |

Compositions A-D were transparent paste or gel-like products. Composition E was not transparent.

Example II

Silicone-containing hair treatment products which deliver high conditioning benefits (as measured by combability) help to prevent hair damage and split end formation. In-vitro experiments were carried out to evaluate the spreadability and wet and dry combing properties of compositions in accordance with the present invention compared with known commercial products which claim to condition hair, smooth down cuticles, seal and prevent split ends. The products tested were as follows:

1. Sebastian International Laminates - 100% silicone/mineral oil mixture containing cyclomethicone, isohexadecane, dimethicone, octyl methoxycinnamate, propyl paraben.
2. L'Oreal Elseve Concentre Special Pointes - containing 95% silicone (made up of a mixture of high and low molecular weight polydimethylsiloxane and cyclomethicone) and ca. 1-2% water and ethanol.
3. Water-in-oil HIPE gel according to the present invention (Formulation A of Example I).

a) Spreadability of wet and dry hair switches.

Method:

Hair switches (10 g/10 in.) were prepared from Yugoslavian dark brown hair. Each switch was washed with 2 x 0.5g of a non-conditioning shampoo and excess water removed by sliding the fingers down the full length of the switch. The switch was then clamped down at both ends on a flat metal surface. (Four switches can be clamped down at one time.)

When measuring spreadability on dried hair, the switch was first dried overnight and combed before clamping down.

0.1 g of oil or 0.5 g of gel was applied 1 cm from the top of the switch. (The HIPE gel contained approx. 20% oil phase; therefore to ensure that an equal amount of oil was delivered on the switch, 0.5 g of gel was applied.) In order to spread the product down the switch evenly, a glass rod that covered all four clamped switches was rolled down the switches. The distance each product was spread down the switch was measured. The results are as follows:

| PRODUCT | DISTANCE FROM TOP OF SWITCH WHERE PRODUCT WAS APPLIED (cm) (mean +/- s.d., n = 5) | |
|---|---|---|
| | Wet hair | Dry hair |
| 1 | 9.0 +/- 0.7 | 9.6 +/- 0.5 |
| 2 | 9.4 +/- 0.5 | 10.0 +/- 0.8 |
| 3 | 14.2 +/- 1.6 | 14.6 +/- 0.9 |

It was noted that the gel in accordance with the invention was easier to spread and gave even coverage down the switch.

b)Ease of combing when applied to wet and dry hair.

Method:

Hair switches (10 g/10 in.) were prepared from Yugoslavian dark brown hair. Each switch was washed with 2 x 0.5g of 16% SLES.2EO and the time taken to untangle the hair was measured; the reading gave the base measurement for that switch. (The switch was combed starting at the bottom, easing the tangles out and moving in small steps up to the top of the switch. The Total Combing Time (TCT) was recorded by means of an electronic timer automatically actuated by the comb.) The switch was then washed with 2 x 0.5g of a non-conditioning shampoo and excess water removed by sliding the fingers down the full length of the switch. The switch was then clamped down at both ends on a flat metal surface.

When measuring ease of combing after applying the product on dried hair, the switch was first dried overnight before clamping down.

0.1 g of oil or 0.5 g of gel was applied 1 cm from the top of the switch. In order to spread the product down the switch evenly, a glass ros was rolled down the switch. The time taken to untangle the hair after product application was measured and compared against the base reading:

$$\%\ TCT = \frac{Product\ TCT}{Base\ TCT} \times 100$$

Thus, the lower the figure for % TCT, the higher the conditioning benefit and also, in this case, a measure of spreadability of the product.

The ease of combing results were as follows:

| PRODUCT | % TCT (mean +/- s.d., n = 3) Product applied on Wet hair | Dry hair |
|---|---|---|
| 1 | 62.8 +/- 2.0 | 34.3 +/- 1.9 |
| 2 | 47.1 +/- 1.0 | 27.8 +/- 1.3 |
| 3 | 38.5 +/- 1.6 | 23.5 +/- 0.5 |

After measuring the total combing time required to untangle the hair switch, the products were left to dry. The switches were then presented to a panel of assessors, the order of presentation being governed by a matrix design to minimise bias. The assessors were asked to comb the switches and judge which was the easiest to comb. The results of this sensory assessment are as follows:

Products applied on wet hair

Least --------------------→ Most easy to comb
Product 1 ~ Product 2 < Product 3
(No. of votes: 23 31 54

Products applied on dry hair

Least --------------------→ Most easy to comb
Product 2 ~ Product 1 < Product 3
(No. of votes: 26 26 56

**Claims**

1. A hair treatment composition comprising a water-in-oil emulsion, wherein the water phase constitutes 40-95% by weight of the composition and the oil phase 5-60% by weight of the composition, wherein the oil phase comprises from 1-100% based on the weight of the oil phase of a silicone material having a viscosity of $10^4$ to $10^9$ mPa.s at 25°C.

2. A hair treatment composition according to claim 1, further comprising up to 20% based on the weight of the oil phase of an emulsifying agent.

3. A hair treatment composition according to claim 2, wherein the emulsifying agent is selected from the group consisting of mono-, di- or tri-phosphoric esters of fatty acids, ethers of glycerol and fatty alcohols, esters of glycerol and fatty acids, ethers of polyethoxylates and fatty acids, sorbitan fatty acid esters and silicone surfactants.

4. A hair treatment composition according to claim 3, wherein the emulsifying agent is a silicone surfactant which is a polymer of dimethyl polysiloxane with polyoxyethylene and/or polyoxypropylene side chains, having a molecular weight of from 10,000 to 50,000 and having the structure:

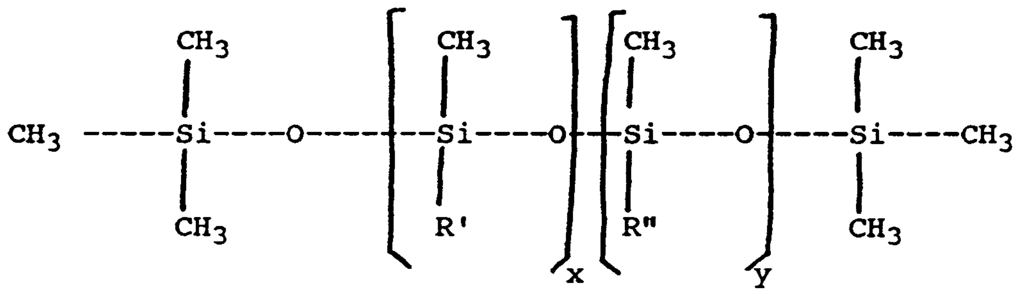

wherein the groups R′ are each chosen from -H, $C_{1-18}$ alkyl and R″ is

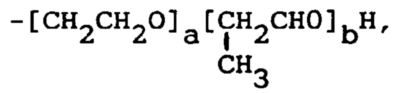

in which
a has a value of from 9 to 115,
b has a value of from 0 to 50,
c has a value of from 133 to 673,
d has a value of from 25 to 0.25.

**5.** A hair treatment composition according to any preceding claim, wherein the silicone material is selected from the group consisting of polyalkyl siloxanes, polyalkylaryl siloxanes, amino functional silicones, polydiorganosiloxanes and mixtures thereof.

**6.** A hair treatment composition according to claim 5, wherein the silicone material is a silicone gum.

**7.** A hair treatment composition according to any preceding claim, wherein the oil phase comprises up to 99% by weight of a carrier or diluent material.

**8.** A hair treatment composition according to claim 7, wherein the carrier or diluent material is selected from the group consisting of cyclomethicones, linear dimethicones, isoparaffins, low viscosity silicones, liquid paraffins or methicones, long chain alkyl alkanoic esters.

**9.** A hair treatment composition according to any preceding claim, wherein the aqueous phase comprises from 20-100% by weight of water.

**10.** A hair treatment composition according to any preceding claim, wherein the aqueous phase comprises up to 75% by weight of a liquid water-miscible material.

**11.** A hair treatment composition according to claim 10, wherein the liquid water-miscible material is selected from the group consisting of lower alcohols, polyols, polyethers, ethoxylated polyols, block copolymers of ethylene oxide and propylene oxide.

**12.** A hair treatment composition according to claim 11, wherein the liquid water-miscible material is glycerol.

**13.** A hair treatment composition according to any preceding claim, wherein the aqueous phase comprises up to 25% by weight of an electrolyte material.

**14.** A hair treatment composition according to any preceding claim, which is in the form of a gel having a viscosity of 1,000 to 100,000 mPa.s at 25°C.

**15.** A method of treating hair including split-ends, comprising applying to recently washed and optionally dried hair a hair treatment composition according to any preceding claim.

European Patent Office

# EUROPEAN SEARCH REPORT

Application Number

EP 91 31 1344

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 271 925 (REVLON)<br>* Whole document *<br>--- | 1-12 | A 61 K 7/06 |
| Y | EP-A-0 323 739 (SUNSTAR K.K.)<br>* Whole document *<br>--- | 1-12 | |
| Y | US-A-4 973 476 (KRZYSIK)<br>* Whole document *<br>--- | 1-12 | |
| X,P | EP-A-0 412 710 (PROCTER & GAMBLE CO.)<br>* Whole document *<br>--- | 1-15 | |
| A | EP-A-0 335 777 (L'OREAL)<br>* Claims; examples *<br>--- | 1-15 | |
| A | US-A-4 834 972 (CHANG)<br>* Whole document *<br>----- | 1-15 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 10-03-1992 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)